Europäisches Patentamt

(19) European Patent Office

Office européen des brevets

(11) Veröffentlichungsnummer: **0 115 606**
**B1**

# (12) EUROPÄISCHE PATENTSCHRIFT

(45) Veröffentlichungstag der Patentschrift:
09.03.88

(51) Int. Cl.⁴: **C 12 P 7/46**

(21) Anmeldenummer: **83112681.8**

(22) Anmeldetag: **16.12.83**

(54) Verfahren zur Gewinnung von L-Äpfelsäure.

(30) Priorität: **24.12.82 DE 3247981**

(43) Veröffentlichungstag der Anmeldung:
**15.08.84 Patentblatt 84/33**

(45) Bekanntmachung des Hinweises auf die Patenterteilung:
**09.03.88 Patentblatt 88/10**

(84) Benannte Vertragsstaaten:
**AT BE CH DE FR GB IT LI LU NL SE**

(56) Entgegenhaltungen:
**DE - A - 2 450 137**
**FR - A - 1 290 212**

**CHEMICAL ABSTRACTS, Band 62, Nr. 13, 21. Juni 1965,
Spalte 16922d, Columbus, Ohio, US
CHEMICAL ABSTRACTS, Band 86, Nr. 3, 17. Januar
1977, Seite 311, Nr. 15232b, Columbus, Ohio, US**

(73) Patentinhaber: **Degussa Aktiengesellschaft,
Weissfrauenstrasse 9, D-6000 Frankfurt am Main 1 (DE)**

(72) Erfinder: **Karrenbauer, Michael, Dr., Dipl.-Chem.,
Lindenstrasse 10, D-6458 Rodenbach (DE)**
Erfinder: **Kleemann, Axel, Dr., Dipl.-Chem.,
Greifenhagenstrasse 25, D-6450 Hanau 9 (DE)**
Erfinder: **Leuchtenberger, Wolfgang, Dr., Dipl.-Chem.,
Geschwister Scholl-Strasse 1, D-6454 Bruchköbel (DE)**
Erfinder: **Moerck, Rudi, Dr., Dipl.-Chem.,
Spessartstrasse 6, D-6460 Geinhausen-Meerholz (DE)**

**Beschreibung**

Die Erfindung betrifft ein Verfahren zur Gewinnung reiner wässriger Lösungen der L-Äpfelsäure aus den bei der enzymatischen Umsetzung von Fumarsäure zu L-Äpfelsäure anfallenden Umsetzungsgemischen unter Verwendung von Ionenaustauschern.

Es ist bekannt, L-Äpfelsäure durch enzymatische Umsetzung von Fumarsäure zu erzeugen, und zwar wird die Fumarsäure im allgemeinen als Salz in wässriger Lösung eingesetzt, so dass die anfallenden Umsetzungsgemische die L-Äpfelsäure und nicht umgesetzte Fumarsäure als Salze enthalten. Es ist ausserdem bekannt, aus diesen Umsetzungsgemischen zwecks Gewinnung reiner L-Äpfelsäure zunächst durch Ansäuern die nicht umgesetzte Fumarsäure abzuscheiden, dann die L-Äpfelsäure als Calciumsalz in Form des Dihydrats abzutrennen, weiter dieses Salz mittels Schwefelsäure zu lösen, die Lösung zwecks Entfernung der Calcium- und der Sulfationen nacheinander über einen sauren und einen basischen Ionenaustauscher zu führen und schliesslich die L-Äpfelsäure abzuscheiden (DE-OS 2 450 137). Das Verfahren ist umständlich und aufwendig, und die Ausbeute an L-Äpfelsäure ist unbefriedigend.

In der JP-A-4 613 (1965) (C.A. 62, 16 922d) wird die fermentative Umsetzung von Di-Natriumfumarat beschrieben. Die Abtrennung des gebildeten Malats erfolgt durch Ausfällen des entsprechenden Calciumsalzes nach der Reinigung der Fermentationsbrühe über Aktivkohle und sauren Ionenaustauschern.

Die FR-A-1 290 212 betrifft die Reinigung von Lösungen organischer Säuren, die man durch Fermentation z.B. von Melasse gewonnen hat.

Mit Hilfe von Kationenaustauschern gelingt die Entfärbung und gleichzeitig die Entfernung der Amingruppen-haltigen Säuren und der Kationen.

Es ist nun ein Verfahren zur Gewinnung reiner wässriger Lösungen der L-Äpfelsäure aus den bei der enzymatischen Umsetzung von Fumarsäure zu L-Äpfelsäure anfallenden Umsetzungsgemischen unter Verwendung von Ionenaustauschern gefunden worden, das dadurch gekennzeichnet ist, dass a) das Umsetzungsgemisch bei Temperaturen etwa zwischen 50 und 150 °C mit einem Kationenaustauscher behandelt und b) das Eluat nach Konzentrierung auf 30 bis 80 Gewichts-% Gehalt an L-Äpfelsäure filtriert wird. Hierbei werden in wenigen einfachen Verfahrensschritten wässrige Lösungen sehr reiner L-Äpfelsäure in hervorragender Ausbeute gewonnen.

Das erfindungsgemässe Verfahren ist zur Gewinnung reiner wässriger Lösungen der L-Äpfelsäure aus den Umsetzungsgemischen geeignet, die bei den üblichen Umsetzungen der Salze der Fumarsäure mittels Enzymen anfallen. Die Umsetzungsgemische enthalten im allgemeinen etwa 5 bis 15 Gewichts-% Salze der L-Äpfelsäure.

Zur Durchführung des erfindungsgemässen Verfahrens werden die Umsetzungsgemische nach Trennung von den Enzymen und den etwaigen ungelösten Bestandteilen mit einem Kationenaustauscher behandelt. Als Kationenaustauscher dient ein üblicher saurer, vorzugsweise ein stark saurer, Ionenaustauscher, beispielsweise auf Polystyrol- oder Polystyrolbenzol-Grundlage, insbesondere ein solcher mit freien Sulfonsäure-Gruppen.

Die Behandlung mit dem Kationenaustauscher erfolgt bei Temperaturen etwa zwischen 50 und 150 °C, vorzugsweise zwischen 70 und 120 °C, insbesondere zwischen 70 und 90 °C. Der Druck kann hierbei weitgehend beliebig gewählt werden, es ist jedoch im allgemeinen zweckmässig, Drücke etwa zwischen dem Normaldruck und einem Druck zu wählen, der bis 3 bar, vorzugsweise bis 1 bar, über dem Dampfdruck der Flüssigkeit bei der angewendeten Behandlungstemperatur liegt.

Je Mol eingesetzten Salzes der Fumarsäure werden mindestens 2 Äquivalente, zweckmässigerweise 2,5 bis 3,0 Äquivalente, des Kationenaustauschers eingesetzt. Die Regenerierung des Ionenaustauschers erfolgt mit starken Säuren, vorzugsweise mit starken Mineralsäuren, insbesondere mit konzentrierter wässriger Chlorwasserstoffsäure.

Das nach der Behandlung mit dem Kationenaustauscher verbleibende Eluat wird durch Verdampfen von Wasser so weit konzentriert, dass der Gehalt an L-Äpfelsäure etwa 30 bis 80 Gewichts-%, vorzugsweise 50 bis 60 Gewichts-%, beträgt.

Das Verdampfen des Wassers erfolgt zweckmässigerweise bei vermindertem Druck bei Temperaturen bis zu etwa 80 °C. Die so konzentrierte Lösung wird bei Temperaturen von etwa 0 bis 20 °C filtriert.

Das Filtrat ist eine reine wässrige Lösung der L-Äpfelsäure. Diese Lösung kann für einige Anwendungszwecke unmittelbar weiterverwendet werden. Die reine L-Äpfelsäure kann aus der Lösung durch weiteres Eindampfen, vorzugsweise unter vermindertem Druck, gegebenenfalls durch Eindampfen bis zur Trockne, abgeschieden werden.

In den Fällen, in denen eine besonders reine L-Äpfelsäure benötigt wird, ist es vorteilhaft, die nach der Filtration verbleibende Lösung mit Aktivkohle zu behandeln. Je Liter Lösung werden zweckmässigerweise 5 bis 100 g, vorzugsweise 10 bis 30 g, Aktivkohle angewendet.

Beispiele

Die gewonnene L-Äpfelsäure wurde jeweils auf ihren spezifischen Drehwert $[\alpha]_D^{20}$, $C_3 = 5,5$ in Pyridin, untersucht. Dieser wird in Grad · cm³/dm · g angegeben. Prozentangaben bedeuten Gewichtsprozente.

1. Es wurde ein Umsetzungsgemisch verwendet, das bei der Umsetzung einer Lösung von 96 g des Natriumsalzes der Fumarsäure in 600 ml Wasser mittels Fumarase nach dem Ultrafiltrations-Verfahren gemäss der DE-OS 2 930 070 angefallen war. Das Umsetzungsgemisch enthielt 67,3 g des Natriumsalzes der L-Äpfelsäure neben 35,5 g des Natriumsalzes der Fumarsäure. Es wurde auf 85 °C erwärmt und bei dieser Temperatur im Verlauf von 60 Minuten auf eine auf 85 °C beheizte Austauschersäule von 5,5 cm Durchmesser und 50 cm Höhe gegeben, die 1000 ml eines stark sauren Kationenaustauschers enthielt (Lewatit S 100, H$^+$-Form). Die nutzbare Kapazität des Austauschers entsprach 1,8 val Wasserstoff-Ionen. Das Eluat wirde bei 70 °C und 100 mbar so weit eingedampft, dass der Gehalt an L-Äpfelsäure auf 32% stieg, und dann im Verlauf von 60 Minuten auf 15 °C abgekühlt. Hierbei schied sich Fumarsäure ab. Diese wurde abfiltriert. (Sie wurde mittels Natriumhydroxid in ihr Natriumsalz übergeführt und für einen weiteren Ansatz verwendet.) Das Filtrat enthielt 49,6 g L-Äpfelsäure, entsprechend 98% Ausbeute, bezogen auf die L-Äpfelsäure, die in dem Umsetzungsgemisch enthalten war. Die so gewonnene L-Äpfelsäure-Lösung enthielt 0,13% Fumarsäure, 1,5 ppm Eisen, 4,5 ppm Natrium, weniger als 100 ppm Chlorid und weniger als 100 ppm Sulfat. Die Lösung wurde mit 7,5 g eisenarmer Aktivkohle (Eponit 113 Spezial) versetzt. Die Mischung wurde unter Rühren 60 Minuten auf 60 °C gehalten und dann bei dieser Temperatur filtriert. Das Filtrat wurde bei 60 °C und 15 mbar zur Trockne eingedampft. Hierbei wurden 49,1 g L-Äpfelsäure gewonnen, entsprechend 97% Ausbeute, bezogen auf die L-Äpfelsäure, die in dem Umsetzungsgemisch enthalten war. Die L-Äpfelsäure war farblos. Sie enthielt 0,09% Fumarsäure, 5 ppm Eisen, 140 ppm Natrium, weniger als 100 ppm Chlorid und weniger als 100 ppm Sulfat. Ihr Schmelzpunkt war 102 bis 104 °C, ihr Drehwert −28,8°.

2. Es wurde wie nach Beispiel 1 verfahren, jedoch wurde das Eluat so weit eingedampft, dass der Gehalt an L-Äpfelsäure auf 56% stieg, und es wurde dann bei 4 °C filtriert. Das Filtrat enthielt 48,7 g L-Äpfelsäure, entsprechend 96% Ausbeute, sowie 0,06% Fumarsäure 2 ppm Eisen, 22 ppm Natrium, weniger als 100 ppm Chlorid und weniger als 100 ppm Sulfat. Durch Eindampfen der Lösung zur Trockne wurden 48,6 g L-Äpfelsäure gewonnen, entsprechend einer Ausbeute von 96%. Die Äpfelsäure enthielt 0,11% Fumarsäure, 4 ppm Eisen, 40 ppm Natrium, weniger als 100 ppm Chlorid und weniger als 100 ppm Sulfat. Ihr Schmelzpunkt war 103 °C, ihr Drehwert −28,8°.

3. Es wurde wie nach Beispiel 1 verfahren, jedoch wurde nach der Behandlung mit dem Kationenaustauscher (Duolite C 26, H$^+$-Form) so weit eingedampft, dass der Gehalt an L-Äpfelsäure auf 65% stieg, und es wurde nach Abkühlung im Verlauf von 12 Stunden bei 0 °C filtriert. Das Filtrat enthielt 47,4 g L-Äpfelsäure, entsprechend 94% Ausbeute, sowie 0,04% Fumarsäure, 2 ppm Eisen, 15 ppm Natrium, weniger als 100 ppm Chlorid und weniger als 100 ppm Sulfat.

4. Es wurde wie nach Beispiel 1 verfahren, jedoch wurde nach der Behandlung mit dem Kationenaustauscher (Duolite C 26, H$^+$-Form) so weit eingedampft, dass der Gehalt an L-Äpfelsäure auf 55% stieg, und es wurde nach Abkühlung im Verlauf von 4 Stunden bei 15 °C filtriert. Das Filtrat enthielt 49,3 g L-Äpfelsäure, entsprechend 97% Ausbeute, sowie 0,2% Fumarsäure, 3 ppm Eisen, 30 ppm Natrium, weniger als 100 ppm Chlorid und weniger als 100 ppm Sulfat. Die Äpfelsäurelösung wurde mit 8,0 g Aktivkohle (Eponit 114 Spezial) versetzt. Die Mischung wurde unter Rühren 60 Minuten lang auf 25 °C gehalten und dann filtriert. Das Filtrat wurde bei 60 °C und 40 mbar zur Trockne eingedampft. Hierbei wurden 49,1 g L-Äpfelsäure gewonnen, entsprechend 97% Ausbeute. Die Äpfelsäure enthielt 0,08% Fumarsäure, 6 ppm Eisen, 55 ppm Natrium, weniger als 100 ppm Chlorid und weniger als 100 ppm Sulfat. Ihr Schmelzpunkt war 101 bis 103 °C, ihr Drehwert −29,1°.

5. Es wurde von einem Umsetzungsgemisch ausgegangen, das bei der Umsetzung von 90 g des Ammoniumsalzes der Fumarsäure in 600 ml Wasser nach dem Ultrafiltrations-Verfahren angefallen war. Das Umsetzungsgemisch enthielt 63,5 g des Ammoniumsalzes der L-Äpfelsäure und 33,3 g des Ammoniumsalzes der Fumarsäure. Die Behandlung mit dem Kationenaustauscher (Duolite C 26 H$^+$-Form) erfolgte bei 85 °C. Das Eluat wurde bei 70 °C und 100 mbar so weit eingedampft, dass der Gehalt an L-Äpfelsäure 55% betrug, und dann im Verlauf von 4 Stunden auf 15 °C abgekühlt. Die nach Abtrennung der Fumarsäure verbliebene Lösung enthielt 49,1 g L-Äpfelsäure, entsprechend 97,0% Ausbeute, 0,2% Fumarsäure und 15 ppm Ammonium. Sie wurde mit 10 g Aktivkohle (Eponit 114 Spezial) versetzt, 60 Minuten lang bei 25 °C gerührt, filtriert und dann bei 60 °C und 40 mbar zur Trockne eingedampft. Es wurden 49,0 g L-Äpfelsäure gewonnen, entsprechend 96,7% Ausbeute. Sie enthielt 0,09% Fumarsäure und 32 ppm Ammonium. Ihr Schmelzpunkt war 101 bis 103 °C, ihr Drehwert −29,1°.

6. Es wurde von einem Umsetzungsgemisch ausgegangen, das bei der Umsetzung von 115 g des Kaliumsalzes der Fumarsäure in 600 ml Wasser nach dem Ultrafiltrations-Verfahren angefallen war. Das Umsetzungsgemisch enthielt 79,4 g des Kaliumsalzes der L-Äpfelsäure und 42,7 g des Kaliumsalzes der Fumarsäure. Im übrigen wurde wie nach Beispiel 5 verfahren. Die gewonnene L-Äpfelsäurelösung enthielt 49,4 g L-Äpfelsäure, entsprechend 97,5% Ausbeute, 0,22% Fumarsäure und 20 ppm Kalium. Die Ausbeute an L-Äpfelsäure betrug 49,3 g, entsprechend 97,3%. Die Äpfelsäure enthielt 0,1% Fumarsäure und 40 ppm Kalium. Ihr Schmelzpunkt war 102 bis 103 °C, ihr Drehwert −29,1°.

7. Es wurde von einem Umsetzungsgemisch ausgegangen, das bei der Umsetzung von 96 g des Natriumsalzes der Fumarsäure in 600 ml Wasser mittels immobilisierter Fumarase angefallen war. Das Umsetzungsgemisch enthielt 74,8 g des Natriumsalzes der L-Äpfelsäure neben 28,8 g des Natriumsalzes der Fumarsäure. Es wurde im Verlauf von 80 Minuten bei 70 °C über 900 ml Kationenaustauscher (Duolite C 26, H$^+$-Form) gegeben. Das Eluat wurde bei 60 °C und 50 mbar so weit eingedampft, dass der Gehalt an L-Äpfelsäure 50% betrug, und dann im Verlauf von 60 Minuten auf 15 °C abgekühlt. Die nach Abfiltrierung der Fumarsäure verbliebene Lösung enthielt 54,6 g L-Äpfelsäure, entsprechend 97% Ausbeute, 0,19% Fumarsäure, 3 ppm Eisen, 40 ppm Natrium, weniger als 100 ppm Chlorid und weniger als 100 ppm Sulfat. Sie wurde bei 60 °C und 50 mbar zur Trockne eingedampft. Es wurden 54,6 g L-Äpfelsäure gewonnen, entsprechend 97% Ausbeute. Die L-Äpfelsäure enthielt 0,39% Fumarsäure, 5 ppm Eisen, 80 ppm Natrium und an Chlorid und Sulfat je weniger als 100 ppm. Ihr Schmelzpunkt war 102 bis 104 °C, ihr Drehwert –28,8°.

8. Es wurde wie nach Beispiel 7 verfahren, jedoch wurde von einem Umsetzungsgemisch ausgegangen, das bei der Umsetzung von 160 g des Natriumsalzes der Fumarsäure in 1000 ml Wasser mittels immobilisierter Fumarase angefallen war und 143,8 g des Natriumsalzes der Äpfelsäure und 30,8 g des Natriumsalzes der Fumarsäure enthielt, und es wurde das Umsetzungsgemisch bei 55 °C über 1700 ml des Kationenaustauschers gegeben. Es wurde eine Lösung gewonnen, die 106 g L-Äpfelsäure, entsprechend 98% Ausbeute, 0,2% Fumarsäure, 2 ppm Eisen und 40 ppm Natrium enthielt.

9. Es wurde wie nach Beispiel 7 verfahren, jedoch wurde das Umsetzungsgemisch bei 120 °C und 1,0 bar Überdruck im Verlauf von 60 Minuten über 1200 ml des Kationenaustauschers gegeben. Die gewonnene Lösung enthielt 54,0 g L-Äpfelsäure, entsprechend 96% Ausbeute, 0,18% Fumarsäure, 4 ppm Eisen und 35 ppm Natrium. Beim Eindampfen der Lösung zur Trockne fielen 53,9 g L-Äpfelsäure an, entsprechend 96% Ausbeute. Diese enthielt 0,36% Fumarsäure, 7 ppm Eisen und 70 ppm Natrium. Ihr Schmelzpunkt war 103 bis 104 °C, ihr Drehwert –28,8°.

## Patentansprüche

1. Verfahren zur Gewinnung reiner wässriger Lösungen der L-Äpfelsäure aus den bei der enzymatischen Umsetzung von Fumarsäure zu L-Äpfelsäure anfallenden Umsetzungsgemischen unter Verwendung von Ionenaustauschern, dadurch gekennzeichnet, dass

a) das Umsetzungsgemisch bei Temperaturen etwa zwischen 50 und 150 °C mit einem Kationenaustauscher behandelt und

b) das Eluat nach Konzentrierung auf 30 bis 80 Gewichts-% Gehalt an L-Äpfelsäure filtriert wird.

2. Verfahren nach Anspruch 1, dadurch gekennzeichnet, dass das Eluat nach der Filtration mit Aktivkohle behandelt wird.

## Claims

1. A process for obtaining pure aqueous solutions of L-malic acid from the reaction mixtures produced during enzymatic conversion of fumaric acid to L-malic acid, using ion exchangers, characterised in that

a) the reaction mixture is treated with a cation exchanger at temperatures of between about 50 and 150 °C and

b) the eluate is filtered after concentration to a 30 to 80% by weight content of L-malic acid.

2. A process according to claim 1, characterised in that the eluate is treated with activated carbon after filtration.

## Revendications

1. Procédé pour l'obtention de solutions aqueuses pures de l'acide L-malique, à partir des mélanges de conversion obtenus dans la conversion enzymatique de l'acide fumarique en acide L-malique, avec utilisation d'échangeurs d'ions, caractérisé en ce que:

a) le mélange de conversion est traité avec un échangeur de cations, à des températures comprises environ entre 50 et 150 °C, et

b) l'éluat est filtré, après concentration à une teneur en acide L-malique allant de 30 à 80% en poids.

2. Procédé selon la revendication 1, caractérisé en ce que, avec la filtration, l'éluat est traité avec du charbon actif.